# FASCICULE DE BREVET EUROPEEN

(11) **EP 1 615 902 B1**
(45) Date de publication et mention de la délivrance du brevet: **20.02.2008**
(21) Numéro de dépôt: 04742484.1
(22) Date de dépôt: 13.04.2004
(51) Int. Cl.: C07D 305/14

(54) **PROCEDE DE PREPARATION DU 4,10 BETA-DIACETOXY-2 ALPHA- BENZOYLOXY-5 BETA 20-EPOXY-1,13 ALPHA-DIHYDROXY-9-OXO-19- NORCYCLOPROPA[G]TAX-11-ENE**
VERFAHREN ZUR HERSTELLUNG VON 4,10 BETA-DIACETOXY-2 ALPHA- BENZOYLOXY-5 BETA, 20-EPOXY-1,13 ALPHA-DIHYDROXY-9-OXO-19- NORCYCLOPROPA[G ]TAX-11-EN
METHOD FOR PRODUCTION OF 4,10 BETA-DIACETOXY-2 ALPHA-BENZOYLOXY-5 BETA,20-EPOXY-1,13 ALPHA-DIHYDROXY-9-OXO-19-NORCYCLOPROPA[G]TAX-11-ENE

(30) Priorité: 14.04.2003 FR 0304613
(43) Date de publication de la demande: 18.01.2006
(73) Titulaire: Aventis Pharma S.A., 92160 Antony (FR)
(72) Inventeur: DIDIER, Eric, F-75013 PARIS (FR); AMOURET, Guy, F-94260 FRESNES (FR)
(86) Numéro de dépôt international: PCT/FR2004/000901
(87) Numéro de publication internationale: WO 2004/092151

(56) Documents cités:
- WO-A-96/32387

## Description

La présente invention concerne un nouveau procédé de préparation de taxoïdes de formule générale :

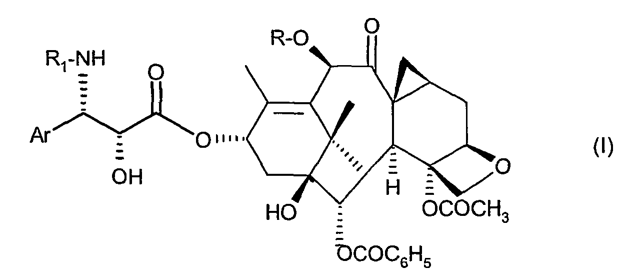

Dans la formule générale (I),
Ar représente un radical aryle,
R représente un atome d'hydrogène ou un radical acétyle, alcoxyacétyle (C3-C6) ou alkyle (C1-C4),
R₁ représente un radical benzoyle ou un radical R₂-O-CO- dans lequel R₂ représente un radical alkyle droit ou ramifié contenant 1 à 8 atomes de carbone.

De préférence Ar représente un radical phényle éventuellement substitué par un ou plusieurs atomes ou radicaux choisis parmi les atomes d'halogène (fluor, chlore, brome, iode) et les radicaux alkyles, alcoxy, alkylthio, cyano, nitro et trifluorométhyle, étant entendu que les radicaux alkyles et les portions alkyles des radicaux contiennent 1 à 4 atomes de carbone, ou bien Ar représente un radical hétérocyclique aromatique ayant 5 chaînons et contenant un ou plusieurs atomes, identiques ou différents, choisis parmi les atomes d'azote, d'oxygène ou de soufre.

Plus particulièrement, Ar représente un radical phényle, thiényle-2 ou -3 ou furyle-2 ou -3.

Plus particulièrement encore, Ar représente un radical phényle éventuellement substitué par un atome de chlore ou de fluor, ou par un radical alkyle (méthyle), alcoxy (méthoxy) ou thiényle-2 ou -3 ou furyle-2 ou -3.

D'un intérêt encore plus particulier est le produit de formule générale (I) dans laquelle Ar représente un radical phényle, R₁ représente un radical tert-butoxycarbonyle et R représente un radical acétyle.

Parmi les procédés connus à ce jour pour préparer les composés de formule (I), on peut citer le brevet EP 0 673 372 qui décrit au départ de 10-déacétylbaccatine III, un procédé qui consiste dans une première étape à protéger en 7 la 10-déacétylbaccatine III, dans une deuxième étape à procéder à une acétylation en position 10, dans une troisième étape à déprotéger la position 7, dans une quatrième étape à effectuer une trifluorométhanesulfonylation (ou triflatation) en position 7 dans une cinquième étape à effectuer une cyclopropylation en position 7-8, puis dans une avant dernière étape à accrocher la chaîne latérale en position 13 et enfin dans une dernière étape à déprotéger la chaîne latérale. L'étape de cyclopropylation en position 7-8 est réalisée en présence soit d'un halogénure de métal alcalin (iodure de sodium, fluorure de potassium) soit d'un azoture de métal alcalin (azoture de sodium) soit d'un sel d'ammonium quaternaire soit enfin d'un phosphate de métal alcalin.

Il est apparu plus tard tel que décrit dans les brevets publiés sous les numéros WO 95/33736, WO 95/33737 et WO 96/32387 que la présence d'azoture ou de d'halogénure de métal alcalin (iodure de sodium, fluorure de potassium) n'était pas indispensable et qu'un additif tel que que les tamis moléculaires en présence de chlorure de sodium était efficace. Le solvant utilisé dans ces trois demandes de brevet était constitué d'un mélange d'acétonitrile et de tétrahydrofurane.

Il est aussi connu d'après l'article de Johnson, Nidy, Dobrowolski, Gebhard, Qualls, Wicnienski et Kelly paru dans Tetrahedron Letters, Vol.35, No 43, pp 7893-7896, 1994 que le 7-O-triflate pouvait être transformé en 7β, 8β-méthano taxane en présence d'un large excès de gel de silice, l'excès est de 60 fois le poids du dérivé 7-O-triflate ce qui est industriellement inutilisable.

Selon la présente invention le procédé décrit dans les trois brevets précités a été amélioré par l'utilisation de sulfolane.

Le composé de formule (I) suivante : 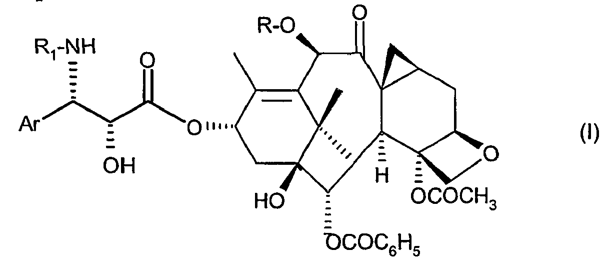 dans laquelle
Ar représente un radical aryle,
R représente un atome d'hydrogène ou un radical acétyle, alcoxyacétyle ou alcoyle,
R1 représente un radical benzoyle ou un radical R2-O-CO- dans lequel R2 représente un radical alcoyle droit ou ramifié contenant 1 à 8 atomes de carbone est préparé par un procédé consistant à mettre en contact le composé de formule (II) 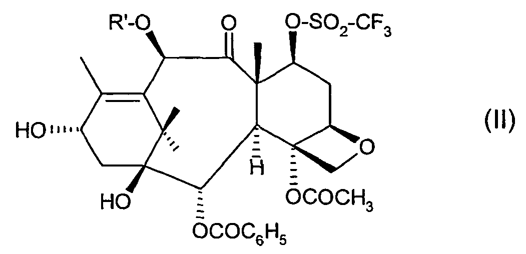 dans lequel R' est un groupe protecteur de la fonction hydroxyle lorsque dans la formule (I) R représente l'hydrogène ou un des groupes cités pour R avec une base faible choisie parmi les tamis moléculaires puis successivement ou préalablement à condenser un précurseur de la chaîne latérale et enfin à déprotéger les fonctions hydroxyles éventuellement protégées caractérisé en ce que la réaction de cyclopropanation a lieu dans le sulfolane.

La présente invention concerne aussi un procédé de préparation d'un intermédiaire de formule générale (III) 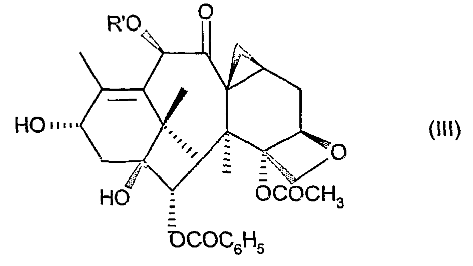 dans laquelle R' a la même signification que dans la formule (II) ci-dessus par mise en contact du dérivé de formule générale (II) avec un tamis moléculaire caractérisé en ce que le procédé est mis en oeuvre dans le sulfolane.

De façon tout à fait préférentielle la réaction de cyclopropylation est effectuée dans le sulfolane contenant entre 2 et 5 % en poids d'eau. La présence d'eau permet la transformation du dérivé de formule (IV) ci-après produit secondaire réalisé lors de la cyclopropanation en dérivé de formule (V), la séparation du dérivé de formules (III) et du dérivé de formule (IV) est difficile, alors que la séparation du dérivé de formule (V) du dérivé de formule (III) est plus facile

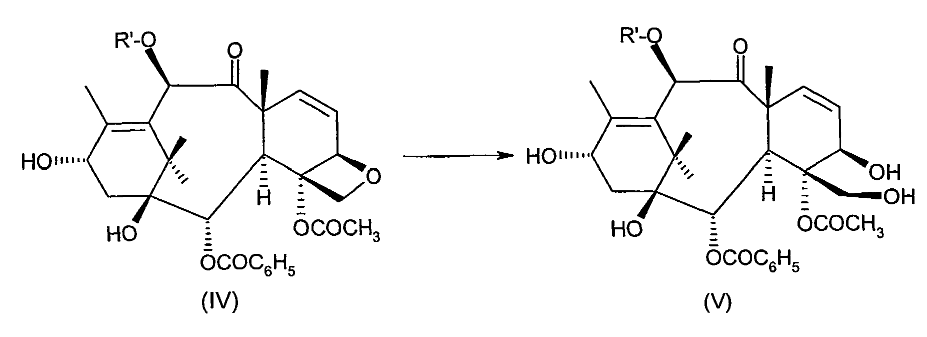

Pour une meilleure mise en oeuvre de l'invention on préfère travailler avec une quantité d'eau d'environ 4 % en poids par rapport au sulfolane. La température réactionelle est notamment comprise entre 20°C et la température d'ébullition du mélange réactionnel.

On ajoute de préférence 25 à 100 % en poids de tamis moléculaire par rapport au substrat. En ce qui concernent les conditions réactionnelles on opère généralement entre 20°C et la température d'ébullition du solvant pendant une à plusieurs heures.

Selon un meilleur procédé de mise en oeuvre de l'invention, on utilisera le sulfolane hydraté (environ 4 % d'eau en poids) comme solvant de réaction en présence de 100 % en poids de tamis moléculaire 4 Å en poudre activé par rapport au substrat. Dans ce cas, la réaction sera réalisée à une température voisine de 60°C jusqu'à transformation complète du substrat.

Le produit de formule (II) dans laquelle R' représente un groupe protecteur de la fonction hydroxyle ou un radical acétyle, alcoxyacéthyle ou alcoyle peut être obtenu par action d'un dérivé de l'acide trifluorométhanesulfonique tel que l'anhydride ou le chlorure sur la baccatine III ou la désacétyl-10 baccatine III protégée sur sa position 10 par un groupe protecteur tel que notamment le trichloroethoxycarbonyle.

Généralement, la réaction d'un dérivé de l'acide trifluorométhanesulfonique s'effectue dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C.

Selon un meilleur procédé de mise en oeuvre de l'invention où on prépare un des composés préférés de formule (III) où R représente un groupe acétyle à partir du composé de formule (II) où R a la même signification après réaction du composé de formule (II) avec le tamis moléculaire :
- on isole le produit brut par une série de traitements tels que l'addition éventuelle d'un solvant tel que l'acétate d'éthyle, l'élimination des insolubles par filtration, la concentration du milieu réactionnel, puis la cristallisation par ajout de solvants insolubilisant tels que choisis notamment parmi l'eau, le toluene.
- puis soit on purifie le produit brut par recristallisation dans un solvant ou mélange de solvant tel que le méthanol, le méthanol en mélange avec le diisopropyléther ou le toluène, le sulfolane en mélange avec le toluène, ou le chlorure de méthylène en mélange avec le diisopropyléther, ou l'acétate d'éthyle en mélange avec le diisopropyléther.
- soit on purifie le produit brut par chromatographie sur gel de silice en éluant avec le chlorure de méthylène en mélange avec l'acétate d'éthyle, le méthanol ou l'acétonitrile.

De préférence, le milieu sera traité par ajout d'acétate d'éthyle, filtration du tamis moléculaire, concentration sous pression réduite et précipitation par ajout d'eau avec ensemencement.

De préférence, le produit brut sera purifiée par cristallisation dans un mélange acétate d'éthyle/diisopropyléther avec un ratio compris entre 50/50 v/v et 10/90 v/v et de préférence égal à 25/75.

Le produit de formule (III) obtenu est ensuite ou préalablement condensé selon des méthodes connues de l'homme de l'art avec un précurseur de la chaine latérale choisi parmi la β-phénylisosérine protégée en position 2' tels que décrit dans le brevet EP 336 840, les oxazolidines tels que notamment décrites dans les brevets EP 595 370, EP 663 906, EP 663 907, EP 663 908, EP 666 857, EP 669 915 ou les β-lactames tels que notamment décrits dans les brevets suivants EP 400 971; US 5,254,580 ; US 5,466,834.

Les exemples suivants illustrent la présente invention, mais ne doivent pas être considérés comme une limitation de l'invention.

### EXEMPLE 1

Dans un tricol de 50 ml, on charge 2,0 g de 4,10β-diacétoxy-2α-benzoyloxy-5β,20-époxy-1,13α-dihydroxy-9-oxo-7β-trifluorométhylsulfonyloxy-tax-11-ène à 92 % de pureté, 2,02 g de tamis moléculaire 4 Å en poudre activé et 1.0 g de chlorure de sodium dans 14 ml de sulfolane et on chauffe à 60°C pendant environ 4 heures. Le milieu réactionnel est refroidi à température ambiante puis filtré. Les insolubles sont lavés en 3 fois avec 50 ml d'acétate d'éthyle et les phases organiques sont rassemblées. La solution de 4,10β-diacétoxy-2α-benzoyloxy-5β,20-époxy-1,13α-dihydroxy-9-oxo-19-norcyclopropa[g]tax-11-ène (I) ainsi obtenue est dosé par CLHP par rapport à un étalon (1,29 g, rendement de 88 %).

### Exemples comparatifs avec différents solvants

Les résultats sont indiqués dans le tableau suivant :

| Exemple | Solvant | Conditions | TT | Rendement | Observations |
|---|---|---|---|---|---|
| C1 | AcCN/THF | 15 g, | >99 % | 69 % | 13,5 % acetamido |
| | 10v/1v | reflux 3 heures | | | 7,7 % ethylen-1 |
| C2 | DMF | 0,5 g | 98 % | 70 % | 16,5 % ethylen-2 |
| | | 40-50°C | | | |
| | | 7 heures 30 | | | |
| C3 | NMP | 0,5 g | > 99% | 12,6 % | Dégradation |
| | | 50°C, 3 heures | | | |
| C4 | Acétone | 3 g | > 99 % | - | 5,6 % ethylen-1 |
| | | reflux 2 heures | | | 40 % 7-epi OH |

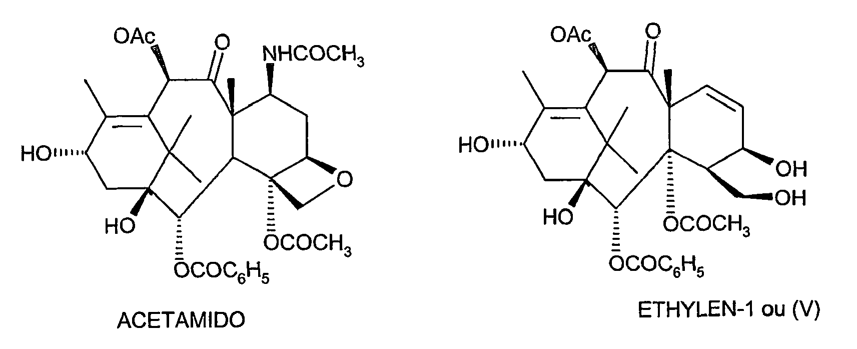

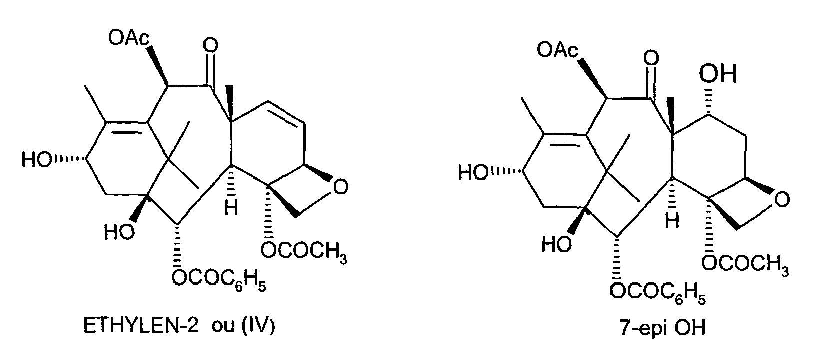

### EXEMPLE 2

Dans un tricol 500 ml, on charge 60,0 g de 4,10β-diacétoxy-2α-benzoyloxy-β,20-époxy-1,13α-dihydroxy-9-oxo-7β-trifluorométhylsulfonyloxy-tax-11-ène à 89,8 % de pureté en poids, 60g de tamis moléculaire 4 Angström en poudre activé et 9,6 ml d'eau dans 240 g de sulfolane et on chauffe sous agitation à 60°C pendant environ 4 heures. Le milieu réactionnel est refroidi à 40°C et on ajoute 200 ml d'acétate d'éthyle. La suspension est filtrée sur un lit de Dicalite et l'insoluble est lavé 4 fois avec 50 ml d'acétate d'éthyle. Les phases organiques sont rassemblées et la solution de 4,10β-diacétoxy-2α-benzoyloxy-5β,20-époxy-1,13α-dihydroxy-9-oxo-19-norcyclopropa[g]tax-11-ène ainsi obtenue est dosé par CLHP par rapport à un étalon (37,5 g, rendement de 88 %). Une fraction de la solution (152 g) est concentrée sous une pression inférieure à 15 mmHg à 45°C pendant environ 45 minutes et le concentrat obtenu (75,2 g) est mis sous agitation à 40°C. On ajoute en une heure 59,2 ml d'eau déminéralisée à la solution puis on ensemence à 40°C avec 100 mg de 4,10β-diacétoxy-2α-benzoyloxy-5β,20-époxy-1,13α-dihydroxy-9-oxo-19-norcyclopropa[g]tax-11-ène. Le milieu est refroidi à température ambiante en 2 heures 30 minutes puis on coule à nouveau en 1 heure 80,3 g d'eau déminéralisée. La suspension est alors refroidie à 0-4°C pendant environ 1 heure 30 minutes. Le produit est filtré, lavé trois fois avec 33 ml d'eau déminéralisée et séché sous pression réduite à 45°C pendant 16 heures. On obtient ainsi 12,65 g de produit brut à 70,8 % de pureté en poids dosée par CLHP (rendement de 83 %).

12,5 g de produit brut sont chargés dans un ballon de 100 ml et dissous dans 25 ml de méthanol à 55°C. Le milieu réactionnel est refroidi à 35°C puis ensemencé avec quelques mg de 4,10β-diacétoxy-2α-benzoyloxy-5β,20-époxy-1,13α-dihydroxy-9-oxo-19-norcyclopropa[g]tax-11-ène. La suspension est alors refroidie à température ambiante puis à 0-4°C pendant environ 3 heures. Après filtration, le produit est lavé deux fois avec 5 ml de diisopropyléther puis séché sous pression réduite à 45°C pendant 16 heures. On obtient ainsi 7,1 g de produit pur à 97,7 % en poids de pureté dosée par CLHP (rendement de recristallisation de 66 %).

### EXEMPLES 3 à 5 On reproduit l'exemple 2 jusqu'au produit brut en ajoutant au sulfolane une quantité d'eau variable

| Exemple | Conditions | ETHYLEN-1 | ETHYLEN-2 |
|---|---|---|---|
| 3 | tamis 60°C, 4 heures | 3,6 | 5,5 |
| 4 | tamis 60°C, 4 heures, 1 % eau | 6,9 | 2,4 |
| 5 | tamis 60°C, 4 heures, 2 % eau | 7,9 | 1,4 |
| 2 | tamis 60°C, 4 heures, 4 % eau | 8,7 | 0,2 |

L'impureté dénommée ETHYLEN-2 est difficile à séparer de (III) par chromatgraphie sur silice ou cristallisation, par contre l'impureté dénommée ETHYLEN-1 s'élimine facilement par ces mêmes techniques.

### EXEMPLE 6 - Purification par cristallisation dans le méthanol

Dans un réacteur en verre de 2 litres, on charge 58 g de 4,10β-diacétoxy-2α-benzoyloxy-β,20-époxy-1,13α-dihydro xy-9-oxo-7β-trifluorométhylsulfonyloxy-tax-11-ène à 92 % de pureté, 58 g de tamis moléculaire 4 Angström en poudre activé et 29 g de chlorure de sodium dans 580 ml d'acétate d'éthyle et on chauffe sous agitation entre 55 et 65°C pendant environ 46 heures. Le milieu réactionnel est refroidi à température ambiante et filtré sur un lit de Clarcel et l'insoluble est lavé 2 fois avec 116 ml d'acétate d'éthyle. Les phases organiques sont rassemblées et la solution de 4,10β-diacétoxy-2α-benzoyloxy-5β,20-époxy-1,13α-dihydroxy-9-oxo-19-norcyclopropa[g]tax-11-ène ainsi obtenue est lavée avec une solution aqueuse d'hydrogénocarbonate de sodium (17,4 g dans 290 ml d'eau) puis deux fois avec 290 ml d'eau. Le milieu réactionnel est concentré jusqu'à un volume d'environ 200 ml sous pression réduite à une température inférieure à 40°C et on ajoute 825 ml de méthanol. Le changement de solvant est effectué par distillation sous pression réduite à une température inférieure à 40°C avec ajout de méthanol (1145 ml au total) puis la solution est refroidie à température ambiante. La cristallisation est amorcée avec 0,21 g de 4,10β-diacétoxy-2α-benzoyloxy-5β,20-époxy-1,13α-dihydroxy-9-oxo-19-norcyclopropa[g]tax-11-ène puis la suspension est refroidie à 0°C pendant environ 1 heure 30 minutes. La suspension est filtrée et le produit est lavé deux fois avec 116 ml de diisopropyléther. Après séchage à température ambiante jusqu'à poids constant, on obtient 25 g de produit (rendement de 55 %) titrant à 94 % en poids par dosage HPLC (> 99 % en normalisation interne de surface)

### EXEMPLE 7 - Purification par chromatographie

4,9 g de 4,10β-diacétoxy-2α-benzoyloxy-5β,20-époxy-1,13α-dihydroxy-9-oxo-19-norcyclopropa[g]tax-11-ène brut à 93,9 % de pureté CLHP (normalisation interne des surfaces) et titrant à 65,5 % en poids sont purifiés par chromatographie avec 250 g de gel de silice en éluant avec un mélange de chlorure de méthylène et d'acétate d'éthyle (v/v : 75/25). Les fractions contenant le produit sont rassemblées et concentrées sous pression réduite. La solution obtenue (241 g) titre à 1,3 % p/p par dosage en CLHP. La pureté par CLHP est de 99,4 % (rendement de purification de 97 %).

### EXEMPLE 8 - Purification par cristallisation dans le diisopropyléther (DIPE)

23 g de 4,10β-diacétoxy-2α-benzoyloxy-5β,20-époxy-1,13α-dihydroxy-9-oxo-19-norcyclopropa[g]tax-11-ène brut à 91 % de pureté par HPLC et titrant à 77,5 % en poids sont mis en solution dans environ 640 ml d'acétate d'éthyle et la phase organique obtenue est lavée deux fois avec 255 ml d'eau. La solution est alors concentrée sous pression réduite à environ 30°C jusqu'à un volume résiduel d'environ 74 ml. 222 ml de diisopropyléther sont alors ajoutés à la solution en 4 à 7 heures à température ambiante puis la suspension obtenue est refroidie à 2°C. La suspension est refroidie et le gâteau de produit est lavé avec 36 ml de diisopropyléther. Après séchage sous pression réduite à 25°C, on obtient 17,6g de 4,10β-diacétoxy-2α-benzoyloxy-5β,20-époxy-1,13α-dihydroxy-9-oxo-19-norcyclopropa[g]tax-11-ène purifié à environ 97 % de pureté et titrant à 89,3 % en poids (rendement de 88 %).

On reproduit l'exemple 8 avec différents solvants de recristallisation.

| Exemple | Conditions | RR % | ETHYLEN-1 % | ETHYLEN-2 % |
|---|---|---|---|---|
| 8 | AcOEt / DIPE | 88 | 0,3 | - |
| | 27/75 | | | |
| 9 | MeOH / DIPE | 82 | 1,7 | 0,2 |
| | 70/30 | | | |
| 10 | MeOH / toluène | 47 | 0,3 | 0,2 |
| | 50/50 | | | |
| 11 | AcOEt/ DIPE | 88 | 0,2 | - |
| | 50/50 | | | |

## Revendications

1. Procédé de préparation du composé de formule (I) 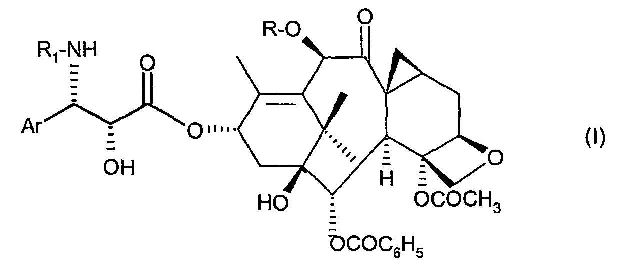 dans laquelle
Ar représente un radical aryle,
R représente un atome d'hydrogène ou un radical acétyle, alcoxyacétyle ou alcoyle,
R₁ représente un radical benzoyle ou un radical R₂-O-CO- dans lequel R₂ représente un radical alcoyle droit ou ramifié contenant 1 à 8 atomes de carbone
par un procédé consistant à mettre en contact le composé de formule (II) 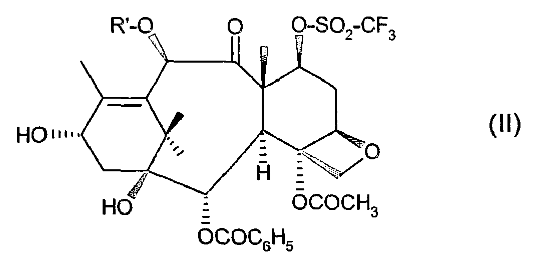 dans lequel R' est un groupe protecteur de la fonction hydroxyle lorsque dans la formule (I) R représente l'hydrogène ou un des groupes cités pour R avec une base faible choisie parmi les tamis moléculaires puis successivement ou préalablement à condenser un précurseur de la chaîne latérale et enfin à déprotéger les fonctions hydroxyles éventuellement protégées **caractérisé en ce que** la réaction de cyclopropanation a lieu dans le sulfolane.

2. Procédé selon la revendication 1 **caractérisé en ce que** R est un radical acétyle, R₁ est un radical terbutoxycarbonyle et Ar est un radical phényle.

3. Procédé selon la revendication 1 **caractérisé en ce que** la réaction est effectuée en présence de tamis moléculaire 4A en poudre activée.

4. Procédé selon les revendications 1 et 3 **caractérisé en ce que** l'on utilise un rapport pondéral entre le tamis moléculaire et le composé de formule (II) d'environ 100 % en poids.

5. Procédé selon les revendications 1 à 4 **caractérisé en ce que** le sulfolane contient 2 à 5 % en poids d'eau.

6. Procédé selon la revendication 5 **caractérisé en ce que** le sulfolane contient environ 4% d'eau.

7. Procédé selon les revendication 1 à 6 **caractérisé en ce que** la température de réaction est comprise entre 20°C et la température d'ébullition du solvant.

8. Procédé selon la revendication 7 **caractérisé en ce que** la température de réaction est d'environ 60°C.

9. Procédé selon la revendication 1 **caractérisé en ce que** le produit brut est isolé par addition d'acétate d'éthyle au milieu réactionnel, filtration du tamis moléculaire, concentration du milieu réactionnel, puis cristallisation par ajout d'eau.

10. Procédé selon la revendication 9 **caractérisé en ce que** après isolement le produit brut est purifié par recristallisation dans un solvant ou mélange de solvant choisi parmi le méthanol, le méthanol en mélange avec le diisopropyléther, le méthanol en mélange avec le toluène, le sulfolane en mélange avec le toluène, le chlorure de méthylène en mélange avec le diisopropyléther, ou l'acétate d'éthyle en mélange avec le diisopropyléther.

11. Procédé selon la revendication 10 **caractérisé en ce que** le produit brut est purifié par un mélange d'acétate d'éthyle et d'isopropyléther selon un ratio d'environ 25/75 v/v.

12. Procédé selon la revendication 1 **caractérisé en ce que** le précurseur de la chaîne latérale est choisi par les dérivés de la β-phénylisosérine protégée en position 2', les oxazolidines ou les β-lactames.

13. Procédé de préparation d'un intermédiaire de formule générale (III) 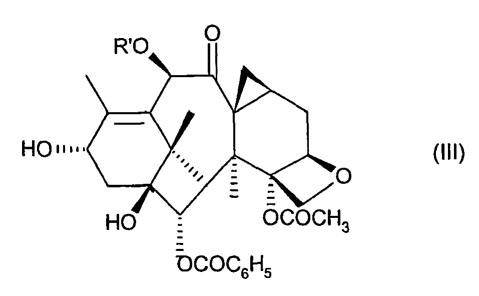 dans laquelle R' a la même signification que dans la formule (II) de la revendication 1
par mise en contact d'un dérivé de formule générale (II) 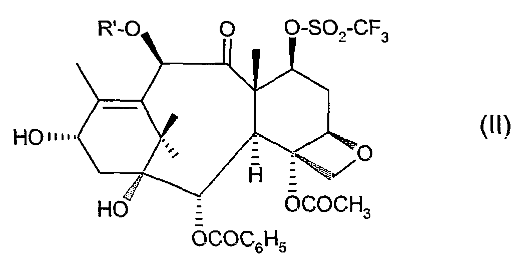 avec un tamis moléculaire **caractérisé en ce que** le procédé est mis en oeuvre dans le sulfolane.

## Claims

1. A process for the preparation of the compound of formula (I) 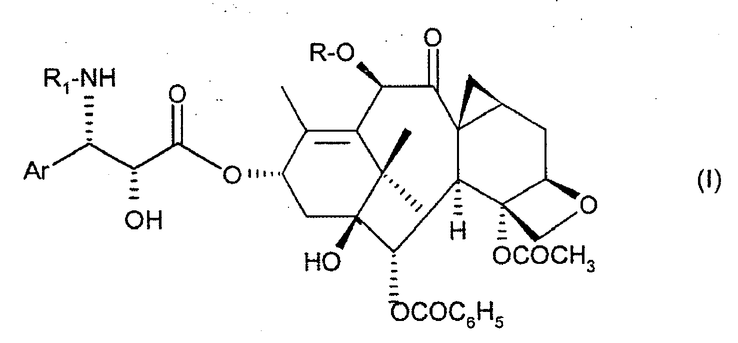 in which
Ar represents an aryl radical,
R represents a hydrogen atom or an acetyl, alkoxyacetyl or alkyl radical,
R₁ represents a benzoyl radical or an R₂-O-CO- radical in which R₂ represents a straight or branched alkyl radical comprising 1 to 8 carbon atoms,
by a process which consists in bringing the compound of formula (II) 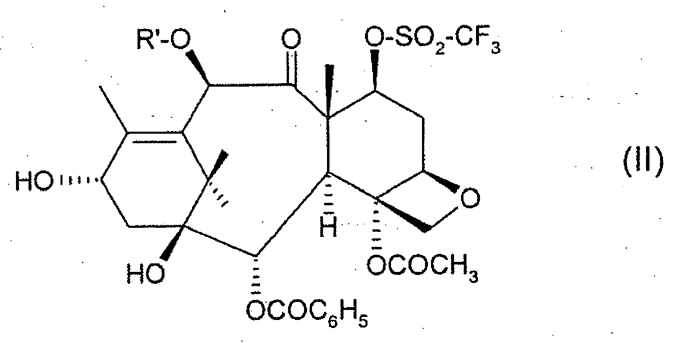 in which R' is a protective group for the hydroxyl functional group when R represents hydrogen in the formula (I) or one of the groups cited for R, into contact with a weak base chosen from molecular sieves, then, successively or beforehand, in coupling a precursor of the side chain and, finally, in deprotecting the optionally protected hydroxyl functional groups, which comprises carrying out the cyclopropanation reaction in sulfolane.

2. The process as claimed in claim 1, wherein R is an acetyl radical, R₁ is a tert-butoxycarbonyl radical and Ar is a phenyl radical.

3. The process as claimed in claim 1, wherein the reaction is carried out in the presence of 4 Å molecular sieve as activated powder.

4. The process as claimed in claims 1 and 3, wherein use is made of a ratio by weight of the molecular sieve to the compound of formula (II) of approximately 100% by weight.

5. The process as claimed in claims 1 to 4, wherein the sulfolane comprises 2 to 5% by weight of water.

6. The process as claimed in claim 5, wherein the sulfolane comprises approximately 4% of water.

7. The process as claimed in claim 1 to 6, wherein the reaction temperature is between 20°C and the boiling point of the solvent.

8. The process as claimed in claim 7, wherein the reaction temperature is approximately 60°C.

9. The process as claimed in claim 1, wherein the crude product is isolated by addition of ethyl acetate to the reaction medium, filtration of the molecular sieve, concentration of the reaction medium and then crystallization by addition of water.

10. The process as claimed in claim 9, wherein, after isolation, the crude product is purified by recrystallization from a solvent or solvent mixture chosen from methanol, methanol as a mixture with diisopropyl ether, methanol as a mixture with toluene, sulfolane as a mixture with toluene, methylene chloride as a mixture with diisopropyl ether, or ethyl acetate as a mixture with diisopropyl ether.

11. The process as claimed in claim 10, wherein the crude product is purified with a mixture of ethyl acetate and isopropyl ether according to a ratio of approximately 25/75 v/v.

12. The process as claimed in claim 1, wherein the precursor of the side chain is chosen by derivatives of β-phenylisoserine protected in the 2' position, oxazolidines or β-lactams.

13. A process for the preparation of an intermediate of general formula (III) 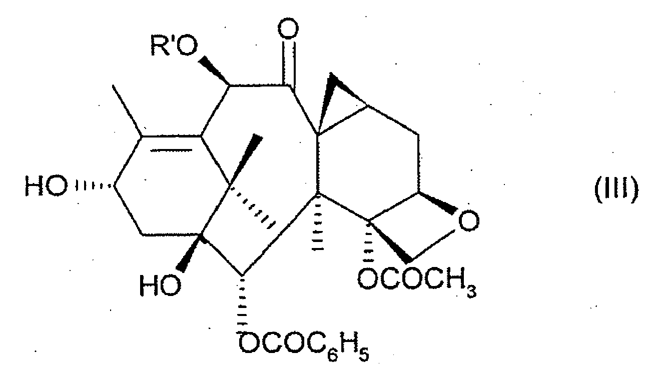 in which R' has the same meaning as in the formula (II) of claim 1,
by bringing a derivative of general formula (II) 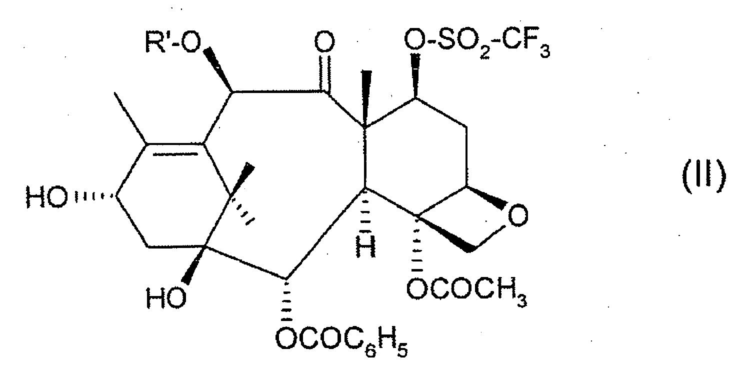 into contact with a molecular sieve, which is carried out in sulfolane.

## Patentansprüche

1. Verfahren zur Herstellung einer Verbindung nach Formel (I) 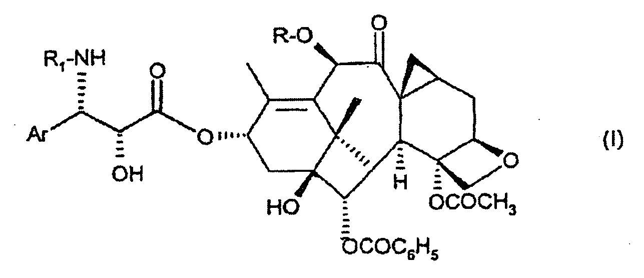 in welcher
Ar für ein Arylradikal steht,
R für ein Wasserstoffatom oder ein Acetyl-, Alkoxyacetyl- oder Alkylradikal steht,
R₁ für ein Benzoylradikal oder ein R₂-O-CO-Radikal, wobei R₂ für ein geradkettiges oder verzweigtes Alkylradikal mit 1 bis 8 Kohlenstoffatomen steht, steht,
mittels eines Verfahrens, das darin besteht, eine Verbindung nach Formel (II) 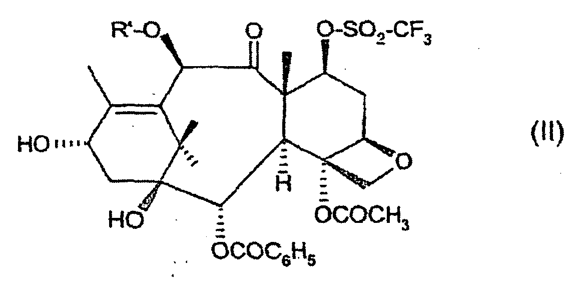 in welcher R' eine Schutzgruppe für die funktionelle Hydroxylgruppe ist, wenn in der Formel (I) R für Wasserstoff oder eine der für R genannten Gruppen steht, mit einer schwachen Base in Kontakt zu bringen, welche aus den Molekularsieben gewählt ist, sowie in einem nachfolgend oder auch im Vorfeld durchgeführten Schritt, durch Kondensation eine Vorläufergruppe der Seitenkette einzuführen, und schließlich die Schutzgruppen von den möglicherweise geschützten funktionellen Hydroxylgruppen zu entfernen, **dadurch gekennzeichnet, dass** die Cyclopropanierungsreaktion in Sulfolan abläuft.

2. Verfahren gemäß Anspruch 1, **dadurch gekennzeichnet, dass** R ein Acetylradikal ist, R₁ ein tert.-Butoxycarbonylradikal ist und Ar ein Phenylradikal ist.

3. Verfahren gemäß Anspruch 1, **dadurch gekennzeichnet, dass** die Reaktion in Gegenwart des aktivierten pulverförmigen Molekularsiebs 4A durchgeführt wird.

4. Verfahren gemäß Ansprüchen 1 und 3, **dadurch gekennzeichnet, dass** das Molekularsieb und die Verbindung nach Formel (II) in einem Gewichtsverhältnis von ungefähr 100 Gew.-% eingesetzt werden.

5. Verfahren gemäß Ansprüchen 1 bis 4, **dadurch gekennzeichnet, dass** das Sulfolan 2 bis 5 Gew.-% an Wasser enthält.

6. Verfahren gemäß Anspruch 5, **dadurch gekennzeichnet, dass** das Sulfolan ungefähr 4 % an Wasser enthält.

7. Verfahren gemäß Ansprüchen 1 bis 6, **dadurch gekennzeichnet, dass** die Reaktionstemperatur zwischen 20 °C und der Siedetemperatur des Lösemittels liegt.

8. Verfahren gemäß Anspruch 7, **dadurch gekennzeichnet, dass** die Reaktionstemperatur ungefähr 60 °C beträgt.

9. Verfahren gemäß Anspruch 1, **dadurch gekennzeichnet, dass** das Rohprodukt isoliert wird, indem Ethylacetat zum Reaktionsmilieu gegeben, das Molekularsieb abfiltriert, das Reaktionsmilieu aufkonzentriert und anschließend durch Wasserzusatz eine Kristallisation bewirkt wird.

10. Verfahren gemäß Anspruch 9, **dadurch gekennzeichnet, dass** das Rohprodukt nach der Isolierung durch Umkristallisation in einem Lösemittel oder einer Mischung aus Lösemitteln, die aus Methanol, Methanol in Mischung mit Diisopropylether, Methanol in Mischung mit Toluol, Sulfolan in Mischung mit Toluol, Dichlormethan in Mischung mit Diisopropylether oder Ethylacetat in Mischung mit Diisopropylether gewählt sind, aufgereinigt wird.

11. Verfahren gemäß Anspruch 10, **dadurch gekennzeichnet, dass** das Rohprodukt durch eine Mischung aus Ethylacetat und Isopropylether nach einem Verhältnis von ungefähr 25/75 (v/v) aufgereinigt wird.

12. Verfahren gemäß Anspruch 1, **dadurch gekennzeichnet, dass** die Vorläufergruppe der Seitenkette aus den Derivaten des β-Phenylisoserins mit Schutzgruppe in Position 2', den Oxazolidinen oder den β-Lactamen gewählt ist.

13. Verfahren zur Herstellung einer Zwischenverbindung nach der allgemeinen Formel (III) 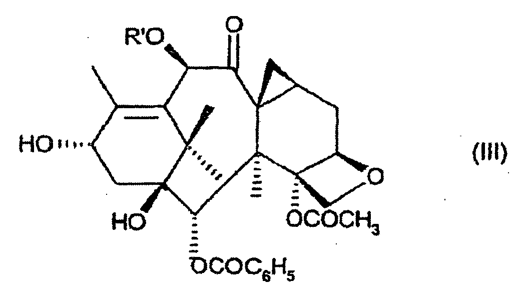 in welcher R' dieselbe Bedeutung wie in der Formel (II) des Anspruchs 1 hat,
indem ein Derivat nach der allgemeinen Formel (II) 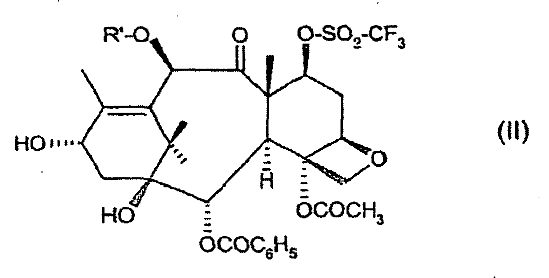 mit einem Molekularsieb in Kontakt gebracht wird, **dadurch gekennzeichnet, dass** das Verfahren in Sulfolan durchgeführt wird.
